# FASCICULE DE BREVET EUROPEEN

(11) **EP 3 022 289 B1**
(45) Date de publication et mention de la délivrance du brevet: **20.02.2019**
(21) Numéro de dépôt: 14790143.3
(22) Date de dépôt: 17.07.2014
(51) Int. Cl.: C12N 1/06, C12R 1/89, A23L 17/60

(54) **PROCÉDÉ OPTIMISE DE RUPTURE DES PAROIS DE CHLORELLES PAR HOMOGÉNÉISATION A TRÈS HAUTE PRESSION**
OPTIMIERTES VERFAHREN ZUM BRECHEN VON CHLORELLA-ZELLWÄNDEN DURCH HOMOGENISIERUNG UNTER SEHR HOHEM DRUCK
OPTIMISED METHOD FOR BREAKING CHLORELLA CELL WALLS BY MEANS OF VERY HIGH PRESSURE HOMOGENISATION

(30) Priorité: 19.07.2013 FR 1357130
(43) Date de publication de la demande: 25.05.2016
(73) Titulaire: Corbion Biotech, Inc., South San Francisco, CA 94080 (US)
(72) Inventeur: PATINIER, Samuel, F-59890 Quesnoy sur Deule (FR)
(74) Mandataire: Cabinet Becker et Associés
(86) Numéro de dépôt international: PCT/FR2014/051839
(87) Numéro de publication internationale: WO 2015/007997

(56) Documents cités:
- EP-A1- 0 222 169
- EP-A1- 2 724 625
- WO-A1-2010/120923
- WO-A2-2010/045368
- CN-A- 103 911 208
- FR-A1- 2 273 064
- SPIDEN ERIN M ET AL: "Quantitative evaluation of the ease of rupture of industrially promising microalgae by high pressure homogenization", BIORESOURCE TECHNOLOGY, vol. 140, 28 avril 2013 (2013-04-28), pages 165-171, XP028565382, ISSN: 0960-8524, DOI: 10.1016/J.BIORTECH.2013.04.074
- NALIN SAMARASINGHE ET AL: "Algal cell rupture using high pressure homogenization as a prelude to oil extraction", RENEWABLE ENERGY, PERGAMON PRESS, OXFORD, GB, vol. 48, 20 avril 2012 (2012-04-20), pages 300-308, XP028428119, ISSN: 0960-1481, DOI: 10.1016/J.RENENE.2012.04.039 [extrait le 2012-05-11]
- DATABASE WPI Week 201215 Thomson Scientific, London, GB; AN 2012-B00362 XP002722081, & CN 102 304 168 A (UNIV SOUTH CHINA TECHNOLOGY) 4 janvier 2012 (2012-01-04)
- DATABASE WPI Week 200943 Thomson Scientific, London, GB; AN 2009-K61199 XP002722082, & CN 101 449 827 A (JIANGXI PINSHENGYUAN BIOLOGY ENG CO LTD) 10 juin 2009 (2009-06-10)
- ERIN M. SPIDEN ET AL: "Critical analysis of quantitative indicators of cell disruption applied to Saccharomyces cerevisiae processed with an industrial high pressure homogenizer", BIOCHEMICAL ENGINEERING JOURNAL, vol. 70, 1 janvier 2013 (2013-01-01), pages 120-126, XP055156038, ISSN: 1369-703X, DOI: 10.1016/j.bej.2012.10.008

## Description

La présente invention est relative à un procédé optimisé de rupture des parois cellulaires des microalgues du genre *Chlorella,* plus particulièrement *Chlorella vulgaris, Chlorella sorokiniana* ou *Chlorella protothecoides* à l'échelle industrielle.

### Présentation de l'état de l'art

Il est bien connu de l'homme du métier que les chlorelles sont une source potentielle de nourriture, car elles sont riches en protéines et autres nutriments essentiels.

Elles renferment notamment 45% de protéines, 20% de matières grasses, 20% de glucides, 5% de fibres et 10% de minéraux et de vitamines.

Pour utiliser efficacement *Chlorella* en Alimentation, il est souvent fait recours au « cassage cellulaire » de manière à faciliter sa digestibilité et son taux d'absorption.

Ce « cassage cellulaire » des microalgues est bien décrit dans la littérature brevets et non brevets par l'emploi de technologies variées :
- physiques (ultrasons, microbilles, chocs thermiques, haute pression...)
- chimiques (acides, alcalis, solvants organiques hydrophiles...)
- enzymatiques (cellulase, lipase...).

Cependant, les différentes alternatives mécaniques, chimiques ou enzymatiques sont généralement peu extrapolables à l'échelle industrielle et essentiellement décrites à l'échelle du laboratoire.

De plus, si la paroi cellulaire présente une résistance mécanique particulièrement élevée (notamment pour le genre *Chlorella*) et si la densité cellulaire dans le milieu est élevé (>100g/L), le choix technologique devient très limité.

Lorsque que l'on intègre en plus les problématiques industrielles d'extrapolation (haute capacité, fiabilité, coûts opérationnels, coûts d'investissement...), les alternatives mécaniques réalistes se restreignent essentiellement au broyage par microbilles et à la technologie de haute pression.

Les technologies classiques de cassage cellulaire par homogénéisation haute pression, domaine auquel s'adresse la présente invention, sont par exemple décrites dans les demandes de brevet ou brevets CN 102433015 (pour l'extraction de cyanine des algues bleues unicellulaires), CN 101817738 (pour l'extraction de DHA à partir de microorganismes en produisant), US 5,8855,64 (pour l'obtention de compositions oxygénées contenant des phospholipides et du fluorocarbone) ou encore CN 101352249 (pour l'extraction des composants lipidiques et protéiques de biomasses de microalgues).

L'homogénéisation à haute pression (dite « HHP »dans la suite de cet exposé), également connue sous le nom homogénéisation à haute pression dynamique, a été proposée à travers le développement d'une nouvelle génération d'homogénéisateurs, capables d'atteindre des pressions de 10-15 fois plus élevé que les machines traditionnelles.

Etant donné la nature du matériel cellulaire à « casser », l'HHP conduit à la production d'émulsions, composées essentiellement d'un mélange de débris cellulaires, de liquide aqueux intracellulaire et d'huile.

Au début des années 1980, une nouvelle technologie a été proposée pour la production d'émulsions fines grâce la disponibilité d'appareils capables de générer et de gérer de très hautes pressions dans les liquides, des pressions supérieures à 100 MPa et jusqu'à 300 - 500 MPa, ainsi que grâce à la nouvelle conception de la chambre d'homogénéisation.

Différents fabricants d'homogénéisateurs haute pression proposent ainsi soit des prototypes, soit de l'équipement à l'échelle industrielle, comme Microfluidics, Stansted Fluid Power, AVP, Avestin ou Niro Soavi.

Dans sa conception, l'homogénéisateur de base se compose d'un générateur à haute pression, telle qu'une pompe à déplacement positif couplée à un amplificateur de pression qui force le fluide à travers un ensemble de soupapes d'homogénéisation spécialement conçues.

La pressurisation rapide du fluide de traitement (jusqu'à 350 MPa) provoque une augmentation de température de l'ordre de 3°C/100 MPa, tandis que la chute de pression instantanée survenant dans la valve d'homogénéisation induit une augmentation de chaleur plus importante (15 à 20 °C/100 MPa).

Une soupape secondaire, où une chute de pression nettement plus faible se produit, peut être placée à côté de la soupape principale afin de perturber les agglomérats éventuellement formés dans la première étape.

Etant donné que la température finale peut être élevée, en fonction de la température d'entrée et du niveau de pression de fonctionnement, un refroidissement rapide du fluide de traitement représente une bonne pratique pour préserver les composants thermolabiles du produit traité.

De façon remarquable, dans ces opérations à haute pression dynamique, le fluide est exposé à de fortes pressions pendant des durées très courtes (1-10 s).

Dans ce laps de temps, le fluide doit s'écouler de l'amplificateur à la soupape de disruption. L'opération de cassage est principalement réglée par le passage du fluide de procédé sous haute pression au travers d'une soupape de décharge à orifice restreint réglable, plutôt que par exposition à haute pression.

Quel que soit le type de valve d'homogénéisation, le liquide traité sous haute pression passe à travers une section convergente appelé « l'espace d'homogénéisation », puis se dilate.

La pression est commandée par un actionneur, ce qui permet de régler la force exercée sur la soupape.

Cette technologie d'HHP demande donc à être finement contrôlée pour être efficace, et d'être adaptée en fonction des objectifs visés.

C'est ainsi qu'à côté de l'utilisation de l'HHP pour rompre la paroi des cellules, par exemple de microalgues afin de les rendre plus digestibles, ou pour en libérer le contenu d'intérêt, une autre application concerne la décontamination microbienne.

Dans ce domaine, la société Stansted Fluid Power Ltd a introduit une amélioration significative à la conception de la valve de l'homogénéisateur, qui permet au moyen de l'augmentation du niveau de pression du fluide jusqu'à 350MPa, de stériliser des produits *in situ* par la modification de la texture des émulsions ou des biopolymères.

Il résulte de tout ce qui précède que la technologie HHP parait prometteuse lorsque l'on vise une extrapolation industrielle efficace de cette technologie de rupture cellulaire.

Cependant, lorsqu'il faut l'appliquer au cassage de cellules de microalgues en général, et de chlorelles en particulier, de nombreuses problématiques se posent.

Une première problématique relative au traitement de la biomasse de Chlorelles par HHP est liée à la nature de l'émulsion produite.

La stabilité de cette émulsion va dépendre, entre autres, des molécules se trouvant à l'interface ainsi que du travail d'émulsification fourni par la technologie d'homogénéisation.

La stabilité de celle-ci est généralement indispensable pour permettre son utilisation dans de multiples applications alimentaires.

Une seconde problématique est relative au séchage de ladite émulsion.

En effet, lorsque cette émulsion est séchée (par exemple, par atomisation), la finesse de l'émulsion va conditionner les propriétés d'écoulement de la poudre, d'encapsulation des lipides, de stabilité face à l'oxydation.

Le produit fini sera d'autant plus facile à mettre en oeuvre selon les applications visées.

Selon la matrice, l'énergie nécessaire à l'émulsification peut être très importante pour parvenir à générer une émulsion suffisamment fine et stable.

Une troisième problématique est la qualité microbienne à respecter lors du traitement de raffinage / purification d'une biomasse humide telle qu'un moût de fermentation de Chlorelles.

Dans le cas où le protocole de raffinage / purification est effectué dans un environnement non stérile, des précautions particulières sont à prendre pour limiter le développement d'une contamination microbienne (par exemple procédé réfrigéré, limitation de la durée des phases de stockage intermédiaires, etc....).

Cependant, ces moyens de prévention peuvent être insuffisants et une étape de pasteurisation / stérilisation peut s'avérer nécessaire parmi les dernières étapes générant le produit fini.

Face à ces trois problématiques, l'homme du métier sera contraint d'avoir recours à un procédé lourd avec successivement les étapes de broyage, de pasteurisation/stérilisation et d'homogénéisation avec les technologies adéquates.

### Objet de l'invention

Pour remédier à ces contraintes, la société Demanderesse a choisi de conduire ses travaux sur la maîtrise de la technologie de très-haute (ou ultra-haute) pression afin de parvenir à effectuer ces trois actions simultanément et ainsi simplifier significativement l'enchainement des opérations.

Plus particulièrement, la société Demanderesse a décidé de tirer profit de deux des différents domaines d'application de la technologie d'HHP, classiquement considérés séparément par l'homme du métier.

Le premier domaine se rapporte aux changements physiques qui peuvent être provoqués par la technologie HHP, tels que la réduction de la taille et le rétrécissement de la distribution de taille des particules, des gouttelettes ou des micelles de suspensions ou d'émulsions, généralement décrits et utilisés pour la préparation ou la stabilisation d'émulsions ou de préparation de type nanoparticules et nano-suspensions, ou en vue d'obtenir de changements de viscosité et de texture.

Le deuxième domaine est axé sur l'effet de « perturbation » cellulaire induite par l'HHP, qui est généralement appliqué à la récupération de matériel intracellulaire dans l'industrie biotechnologique et pharmaceutique, ou généralement l'HHP est utilisé pour réduire la charge microbienne dans les produits alimentaires et pharmaceutiques.

Cette conduite de l'HHP permet ainsi de l'utiliser efficacement seule pour casser les cellules de microalgues, en tirant profit simultanément de toutes les fonctionnalités qu'offre cette technologie, afin de gérer les problèmes afférents à :
- la production d'une émulsion,
- le séchage de ladite émulsion
- la présence de contaminants microbiens potentiels lors des étapes de raffinage / purification de la biomasse lysée.

Elle permet également de s'affranchir de la nécessité de coupler l'HHP avec d'autres technologies, tel que décrit dans l'état de l'art (comme celle mettant en oeuvre des enzymes spécifiques de dégradation des parois cellulaires de microalgues) pour casser efficacement les cellules de microalgues.

Ainsi, la présente invention est relative à un procédé de cassage de paroi cellulaire, à l'échelle industrielle, de cellules de microalgues du genre *Chlorella,* le cassage de paroi cellulaire étant conduit par homogénéisation à haute pression, caractérisé en ce que l'homogénéisation haute pression est conduite :
- en au moins une passe à une pression entre 300 et 400 MPa, ou
- en au moins deux passes successives à une pression comprise entre 150 et 300 MPa, et
- à une température d'alimentation comprise entre 4 et 40°C,
de manière à garantir un taux de cassage de paroi cellulaire de plus de 80%, une granulométrie de l'émulsion produite inférieure à 1 µm et une diminution de la charge microbienne, notamment par un facteur 1000 ou 10000.

En particulier, le procédé garantit une charge microbienne de moins de 10 germes totaux par g d'émulsion.

De préférence, l'homogénéisation haute pression est conduite sur une biomasse de cellules de microalgues comprenant entre 15 et 50% en poids de matière sèche, en particulier une biomasse de 15 à 50 % en poids sec de cellules de microalgues.

De préférence, les microalgues du genre Chlorella sont choisies dans le groupe constitué de *Chlorella vulgaris, Chlorella sorokiniana* et *Chlorella protothecoides,* et sont plus particulièrement *Chlorella protothecoides.* De préférence, la biomasse de microalgues comprend au moins 10 % en poids sec de lipides, de préférence au moins 20, 30, 40, 50 ou 60 % en poids sec de lipides.

Dans un mode de réalisation particulier, la température d'alimentation est comprise entre 20 et 40°C.

Dans un mode de réalisation préféré, l'homogénéisation haute pression est conduite à une pression entre 300 et 400 MPa en une seule passe.

Dans un autre mode de réalisation préféré, l'homogénéisation haute pression est conduite à une pression entre 150 et 300 MPa, de préférence entre 150 et 250 MPa, en deux, trois, quatre ou cinq passes successives. En particulier, l'homogénéisation haute pression est conduite à une pression comprise entre 150 et 250 MPa en deux passes successives. Les pressions de deux passes successives peuvent être identiques ou différentes.

La présente invention est également relative à un procédé de préparation d'une farine de microalgues, de préférence du genre *Chlorella,* en particulier *Chlorella protothecoides,* comprenant la production d'une biomasse de microalgues, le cassage de paroi cellulaire par un procédé selon la présente invention, et le séchage de la biomasse, en particulier par atomisation.

Une farine de microalgues obtenue par le procédé est décrite. De préférence, le procédé comprend au moins deux passes successives à une pression comprise entre 150 et 300 MPa.

Elle concerne enfin l'utilisation du procédé de cassage de paroi cellulaire de microalgues selon la présente invention pour la préparation d'une farine de microalgues telles que décrites ci-dessus.

### Description détaillée de l'invention

La société Demanderesse a trouvé que l'exploitation raisonné de la technologie d'homogénéisation de très-haute (ou ultra-haute) pression pour rompre la paroi de microalgues du genre *Chlorella,* permet de parvenir à la qualité de broyage souhaitée.

Cette exploitation raisonnée s'entend de l'étude et l'optimisation de chacun des paramètres de conduite de l'HHP adaptées à la microalgue modèle, ici *Chlorella protothecoides.*

Les microalgues préférées de l'invention peuvent croître en conditions hétérotrophiques (sur sucres comme source carbonée et en l'absence de lumière). La société Demanderesse recommande de choisir des microalgues du genre *Chlorella* riches en lipides. Les microalgues utilisées peuvent être choisies, et de manière non-exhaustive, parmi les *Chlorella protothecoides, Chlorella kessleri, Chlorella minutissima, Chlorella sp., Chlorella sorokiniama, Chlorella luteoviridis, Chlorella vulgaris, Chlorella reisiglii, Chlorella ellipsoidea, Chlorella saccarophila, Parachlorella kessleri, Parachlorella beijerinkii, Prototheca stagnora et Prototheca moriformis.* De manière préférée, les microalgues utilisées selon l'invention appartiennent à l'espèce *Chlorella protothecoides.*

Les microalgues sont cultivées en milieu liquide pour produire la biomasse en tant que telle. Selon l'invention, les microalgues sont cultivées dans un milieu contenant une source de carbone et une source d'azote en absence de lumière (conditions hétérotrophiques). Les milieux de croissance solides et liquides sont généralement disponibles dans la littérature, et les recommandations pour la préparation des milieux particuliers qui conviennent à une grande variété de souches de microorganismes peuvent être trouvées, par exemple, en ligne à www.utex.org/, un site maintenu par l'Université du Texas à Austin pour sa collection de culture d'algues (UTEX). La production de biomasse est réalisée en fermenteurs (ou bioréacteurs).

Les exemples spécifiques de bioréacteurs, les conditions de culture, et la croissance hétérotrophe et les méthodes de propagation peuvent être combinés de toute manière appropriée pour améliorer l'efficacité de la croissance microalgale et des lipides.

De préférence, la biomasse à laquelle est appliqué le procédé d'homogénéisation de très-haute pression à une matière sèche comprise entre 15 et 50 % en poids sec. En particulier, la teneur en matière sèche peut être comprise entre 25 et 45 %, de préférence entre 35 et 45 %. De préférence, la biomasse est lavée et concentrée préalablement à l'application du procédé d'homogénéisation de très-haute pression. Dans un mode de réalisation préféré, lorsqu'une teneur en matière sèche est mentionnée, elle signifie une teneur en poids sec de cellules de microalgues.

En outre, la teneur en lipides de la biomasse de microalgues est de préférence d'au minimum au moins 10, 20, 30, 40, 50 ou 60 % en poids sec, par exemple entre 20 et 80 % ou entre 30 et 70 %.

### Evaluation du taux de cassage cellulaire en fonction de la pression appliquée, du nombre de passage et de la température du fluide d'alimentation

Comme il sera exemplifié ci-après, on vérifie que la pression appliquée sur le fluide et le nombre de passes ont un impact significatif sur l'efficacité du cassage cellulaire.

Il est alors recommandé, selon le procédé conforme à l'invention, de travailler :
- à une pression entre 300 et 400 MPa en au moins une passe et de préférence en une seule passe, ou
- à une pression comprise entre 150 et 300 MPa, de préférence entre 150 et 250 MPa, en au moins deux passes successives, et
- à une température d'alimentation comprise entre 4 et 40°C

Avec ce procédé, le pourcentage de cassage des parois cellulaires est de plus de 80, 85 ou 90 %. Par ailleurs, la charge microbienne est significativement diminuée par ce procédé. Notamment, elle est de moins de 10 germes totaux par g d'émulsion.

Dans le mode de réalisation à une passe, la pression peut être comprise entre 300 et 325 MPa, entre 325 et 350 MPa, entre 350 et 375 MPa, ou entre 375 et 400 MPa.

Dans le mode de réalisation à plusieurs passes successive, la pression peut être comprise entre 150 et 175 MPa, entre 175 et 200 MPa, entre 200 et 225 MPa, entre 225 et 250 MPa, entre 250 et 275 MPa, ou entre 275 et 300 MPa. Notamment, la pression peut être comprise entre 200 et 300 MPa ou entre 200 et 250 MPa.

La température d'alimentation peut être comprise entre 20 et 40°C. De manière alternative, elle peut être comprise entre 4 et 10°C, entre 10 et 20° C, entre 20 et 30°C ou entre 30 et 40 °C.

### Evaluation de la capacité de la technologie HHP à générer une émulsion de fines particules

Comme il sera montré ci-après, les observations en microscopie optique, avec ou sans coloration, montrent que la technologie de cassage par HHP permet de générer une émulsion plus fine que celle produite par une méthode de cassage cellulaire plus classique, en l'occurrence par broyage à billes.

La fraction émulsion issue du cassage cellulaire HHP est caractérisée par une très faible granulométrie alors que celle issue du broyage à billes est caractérisée par une granulométrie plus grossière de 1 à plus de 40 µm. En effet, avec le procédé selon la présente invention, une population de granulométrie inférieure à 1 µm est significativement présente, et peut même être majoritaire dans l'émulsion.

Dans un mode de réalisation, la fraction émulsion issue du cassage cellulaire HHP obtenue par le procédé selon la présente invention est caractérisée par la granulométrie d'une émulsion préparée avec cette farine. Les émulsions sont ensuite analysées par granulométrie laser (Laser Mastersizer 2000^{E} - Malvern).

Ainsi, l'émulsion obtenue par le procédé selon la présente invention est caractérisée une population de granulométrie inférieure à 1 µm représentant au moins 20, 30, 40 ou 50 % de la population totale. Par % est entendu ici le pourcentage en répartition de surface dans un graphique représentant le % de volume en fonction de la taille des particules.

Dans un mode de réalisation préféré, l'émulsion obtenue par le procédé selon la présente invention est caractérisée par une population de granulométrie inférieure à 1 µm représentant plus de 50 % de la population totale. Par % est entendu ici le pourcentage en répartition de surface dans un graphique représentant le % de volume en fonction de la taille des particules.

Dans un mode de réalisation particulièrement préféré, le procédé comprend au moins deux passes successives à une pression comprise entre 150 et 300 MPa, de préférence entre 200 et 300 MPa, en particulier 250 MPa, et l'émulsion obtenue par le procédé selon la présente invention est caractérisée une population de granulométrie inférieure à 1 µm représentant plus de 50 % de la population totale.

Par sa caractéristique granulométrique, l'émulsion obtenue par homogénéisation haute pression est donc beaucoup plus stable que celle issue du broyage à billes.

Bien qu'en utilisant de très haute pression, notamment au moins 300 MPa, il a été démontré qu'un seul passage était suffisant pour le procédé de cassage des cellules de microalgues, il a été également démontré par la société Demanderesse qu'une homogénéisation à très haute pression conduira à une émulsion d'autant plus stabilisée que l'on la produira en plusieurs passages successifs. Ainsi, le procédé peut comprendre deux, trois, quatre ou cinq passages successifs. Cependant, dans un intérêt industriel, il est préférable de limiter le nombre de passages. Ainsi, dans un mode de réalisation préféré, deux passages successifs seront utilisés. La pression utilisée pour chaque passage peut varier ou être constante.

Le procédé peut être mis en oeuvre sur tout homogénéisateurs haute pression disponibles, par exemple ceux proposés par Microfluidics, Stansted Fluid Power, AVP, Avestin ou Niro Soavi.

La présente invention est également relative à un procédé de préparation d'une farine de microalgues telles que décrites ci-dessus, comprenant la production d'une biomasse de microalgues, le cassage de paroi cellulaire par un procédé selon la présente invention, et le séchage de la biomasse, en particulier par atomisation. Préalablement au cassage de paroi cellulaire, il est possible de laver la biomasse et/ou de la concentrer. La farine de microalgues obtenue par le procédé ci-dessus est décrite.

Plus généralement, la présente invention concerne une méthode de préparation de farine de microalgues telles que décrites ci-dessus mettant en oeuvre le procédé de cassage de paroi cellulaire de microalgues selon la présente invention. Elle concerne donc l'utilisation du procédé de cassage de paroi cellulaire de microalgues selon la présente invention pour la préparation d'une farine de microalgues telles que décrites ci-dessus.

Cette farine de microalgues est utile dans le domaine alimentaire. Ainsi l'utilisation de la farine obtenue par le procédé selon la présente invention dans les domaines alimentaires est décrite Notamment, elle est relative à une méthode de préparation d'une composition alimentaire comprenant l'ajout d'une telle farine de microalgues à des ingrédients de la composition alimentaire ou à la composition alimentaire. De telles utilisations sont par exemple décrites dans les demandes de brevet WO2010/045368, WO2010/120923 ou US2010/0297296.

Par « échelle industrielle » est de préférence entendu un procédé dans lequel :
- le volume de la chambre de broyage ou cassage est supérieur ou égal à 100 litres, de préférence à 500 litres ; et/ou
- le débit est supérieur à 1 m³/h ; et/ou
- un batch de 1 à 200 m³.

En outre, dans un mode préféré d'échelle industrielle, la biomasse présente 15 à 50 % en poids de matière sèche, plus particulièrement en poids sec de cellules.

L'invention sera mieux comprise à l'aide des exemples qui suivent, lesquels se veulent illustratifs et non limitatifs.

### EXEMPLES

### Exemple 1. Préparation d'une biomasse de microalaues Chlorella protothecoides et présentation des outils mis en oeuvre

Le protocole de fermentation est adapté de celui décrit en toute généralité dans la demande de brevet WO 2010/120923.

Le fermenteur de production est inoculé avec une préculture de *Chlorella protothecoides.* Le volume après inoculation atteint 9 000 L.

La source de carbone employée est un sirop de glucose 55 % p/p stérilisé par application d'un barème temps/température.

La fermentation est un « fed-batch » durant lequel le débit de glucose est ajusté de sorte à maintenir une concentration de glucose résiduel de 3 à 10 g/L.

La durée du fermenteur de production est de 4 à 5 jours.

En fin de fermentation, la concentration cellulaire atteint 185 g/L.

Pendant la phase d'alimentation en glucose, la teneur en azote dans le milieu de culture est limitée pour permettre l'accumulation de lipides à hauteur de 50 % (en poids de biomasse).

La température de fermentation est maintenue à 28°C

Le pH de fermentation avant inoculation est ajusté à 6,8 puis est régulé sur cette même valeur pendant la fermentation.

L'oxygène dissous est maintenu à un minimum de 30 % en contrôlant l'aération, la contre pression et l'agitation du fermenteur.

Le moût de fermentation est traité thermiquement sur une zone HTST avec un barème de 1 min à 75°C et refroidi à 6°C.

La biomasse est alors lavée à l'eau potable décarbonatée avec un ratio de dilution de 6 pour 1 (eau/moût) et concentrée à 250 g/L (25% DCW poids sec en cellules « *Dry Cell Weight* ») par centrifugation en utilisant une Alfa Laval FEUX 510.

### Mesure du taux de cassage cellulaire

La mesure du taux de broyage est effectuée par comptage microscopique des cellules résiduelles après broyage relativement à l'échantillon initial de référence.

Les échantillons sont dilués au 1/800.

L'analyse est effectuée par comptage sur cellule de Malassez selon la méthode d'utilisation standard au microscope optique au grossissement 10x40.

Le taux de cassage cellulaire est déterminé par calcul du pourcentage de cellules résiduelles par rapport à l'échantillon initial de référence.

### Exemple 2. Mesure de l'impact des valeurs de pression, du nombre de passages et de la température sur l'efficacité du cassage de Chorella protothecoides par HHP

On teste l'impact de la valeur de la pression appliquée (100 MPa et 150 MPa), et le nombre de passes, sur l'efficacité de broyage, exprimé en % de taux de cassage, de la biomasse préparée selon l'exemple 1.

Un système d'homogénéisation haute pression Rannie LAB 10.51VH homogeniser (SPX) avec une valve SEO est utilisé à une pression dynamique allant jusqu'à 150 MPa. La biomasse est introduite à un débit d'environ 60L/h.

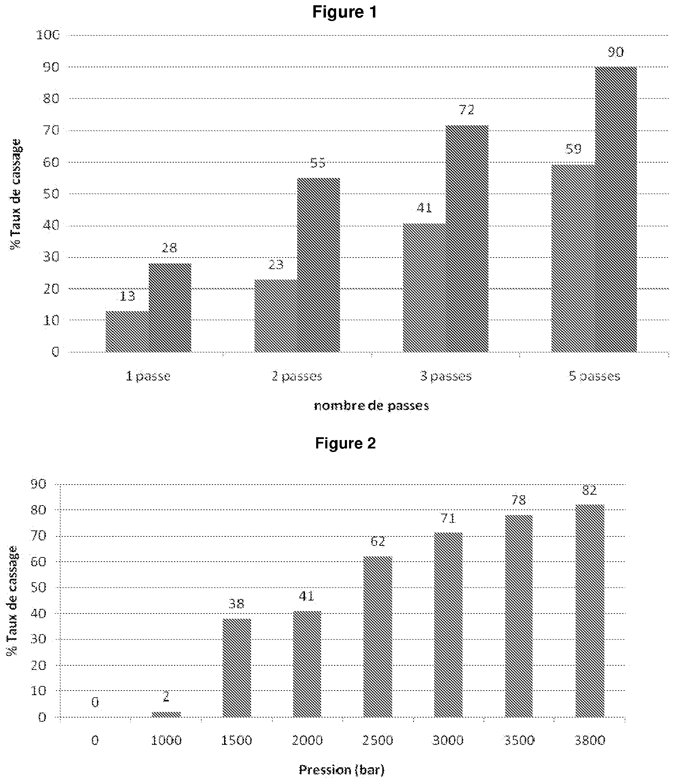

La Figure 1 montre clairement que plus la pression et le nombre de passes est élevé, meilleure sera l'efficacité de cassage.

Une seconde série d'essais est réalisée en un seul passage, mais à différentes pression afin d'évaluer l'efficacité du cassage cellulaire selon la pression appliquée.

Pour ce faire, un système continu d'ultra haute pression Stansted Fluid Power Ltd. 11300 (15 kW) est utilisé à une pression dynamique allant jusqu'à 380 MPa.

Le produit est alimenté de façon continue à un débit d'environ 100 l/h en une seule passe.

Comme le montre la Figure 2, le cassage des cellules est visible à partir de 100 MPa et commence à être significatif à partir de 150 MPa.

Le taux de cassage cellulaire franchit 70% à 300 Mpa et atteint un taux au-delà de 80% à la pression maximale atteignable par la technologie.

Une troisième série d'essais est réalisée en effectuant deux passes successives mais en limitant la pression à 250 Mpa.

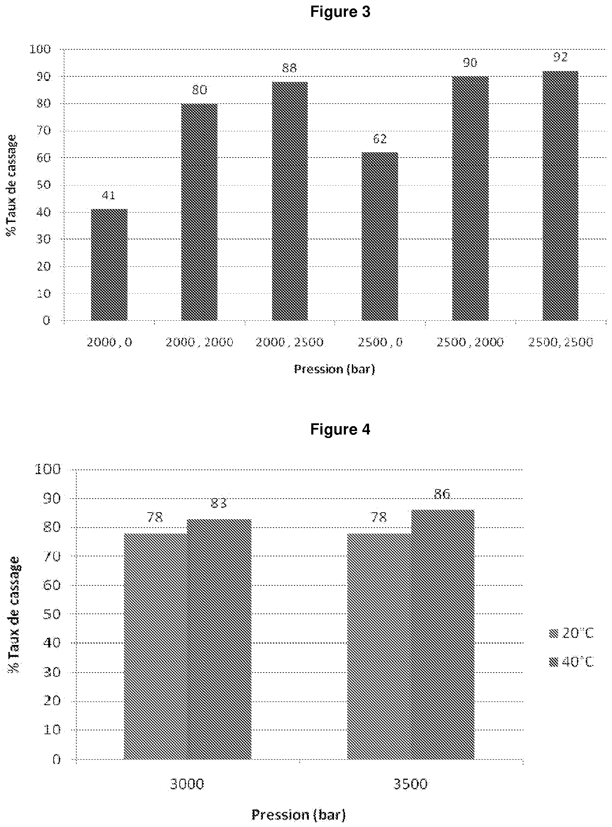

Comme le montre la Figure 3, dans cette configuration, en cumulant deux passes successives à des pressions supérieures à 200 MPa, des taux de cassage cellulaire de l'ordre de 90% sont obtenus.

Dans cette configuration, il est possible de limiter la pression appliqué sur la valve d'homogénéisation à une valeur inférieure à 250 MPa.

Ce schéma permet de limiter les phénomènes d'usure de la valve d'homogénéisation.

L'impact de la température d'entrée sur l'efficacité du cassage cellulaire est évalué dans une autre série d'essais

L'augmentation de la température d'alimentation du produit agit favorablement sur l'efficacité du cassage cellulaire (plus de 5% de gain) - Cf. Figure n°4.

Cependant, l'exothermie du processus d'homogénéisation haute pression génère une augmentation de température très importante pouvant constituer une contrainte selon la sensibilité du produit en sortie, la température d'entrée est donc à limiter selon le seuil maximum tolérable en sortie.

En conclusion : il est recommandé, selon un mode préféré du procédé conforme à l'invention, de travailler :
- à une pression entre 350 et 400 MPa en une seule passe, ou
- à une pression comprise entre 200 et 250 MPa en deux passes successives,
- à une température d'alimentation comprise entre 4 et 40°C

### Exemple 3. Qualité de l'émulsion générée par le cassage HHP

La composition de la biomasse issue de la fermentation selon l'exemple 1 est caractérisée par une fraction lipidique majoritaire (env. 50%/sec).

Après cassage cellulaire, une émulsion (phase aqueuse avec débris cellulaires / huile) est ainsi générée.

La stabilité de cette émulsion est conditionnée par la finesse des globules lipidiques.

L'homogénéisation a pour but de minimiser le diamètre des globules lipidiques et en même temps de le rendre aussi uniforme que possible; il en résulte alors une amélioration de la stabilité et un accroissement de la viscosité du milieu.

La technologie d'homogénéisation haute pression est ainsi évaluée, par rapport à la technologie de broyage à billes, sur son potentiel d'homogénéisation et ainsi de stabilisation de l'émulsion générée au-delà du simple objectif de cassage cellulaire.

La biomasse est alors broyée avec un broyeur à billes NETZSCH Labstar en utilisant des billes de silicate de zirconium de 0,5mm de diamètre (vitesse périphérique : 12 m/s, 90% de taux de remplissage, débit : 6 kg/h)

Une évaluation au microscope optique est ainsi réalisée afin de comparer l'émulsion selon le procédé employé.

Les Figures 5 et 6 permettent de visualiser la différence de taille de l'émulsion générée par les deux technologies (HHP menée en une passe, à 350 Mpa et Broyage à billes).

La Figure 6 met en évidence le fait que le broyage à billes conduit à produire une émulsion beaucoup plus grossière avec une taille de globules plus importante et plus hétérogène que celle obtenue après traitement HHP (Figure 5), l'émulsion générée est visuellement beaucoup plus fine.

Afin de caractériser plus précisément, le potentiel d'émulsification de la technologie HHP en regard de ce qui est obtenu par broyage à billes, les émulsions générées sont analysées sur granulométrie laser (Laser Mastersizer 2000^{E} - Malvern) selon les spécifications du constructeur.

La Figure 7 permet de caractériser l'efficacité des méthodes employées dans la génération d'une émulsion fine et potentiellement stable.

Après cassage cellulaire, dans les deux cas, une population bimodale est observée par rapport à la biomasse initiale qui présente quant à elle une population monomodale, d'un diamètre de cellules entières réparti entre 1 et 10 µm.

Cependant, dans le cas du broyage à billes, conformément aux observations microscopiques, l'émulsion est grossière, répartie entre 1 et 100 µm.

A savoir que la 1^{ère} population, entre 1 et 30 µm est constituée de cellules entières résiduelles ainsi que de globules lipidiques de taille < 30 µm.

La 2^{ème} population est quant à elle constituée d'une émulsion constituée de globules lipidiques de diamètre supérieur à 40 µm.

Globalement, l'émulsion issue du broyage à billes est donc grossière et très hétérogène.

Dans le cas du cassage cellulaire par homogénéisation haute pression, c'est la 2^{ème} population, répartie entre 1 et 10 µm, qui est exclusivement constituée de cellules entières résiduelles.

La fraction émulsion issue du cassage cellulaire est caractérisée comme 1^{ère} population, par une très faible granulométrie, inférieure à 1 µm.

Par sa caractéristique granulométrique, l'émulsion obtenue par homogénéisation haute pression est donc beaucoup plus stable que celle issue du broyage à billes.

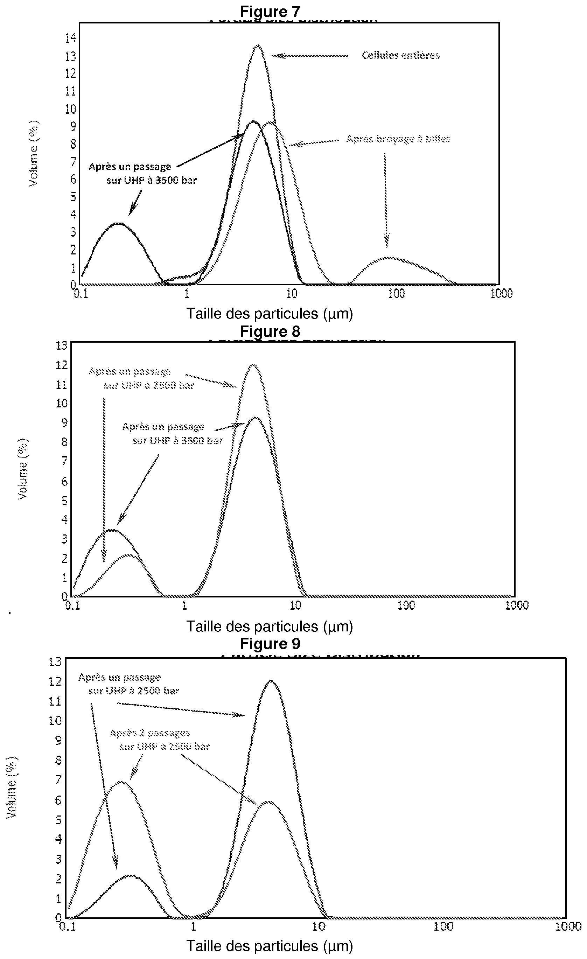

La Figure 8 met en ensuite évidence l'impact de la pression d'homogénéisation sur la répartition granulométrique de l'émulsion.

On note en effet que le taux de cellules entières résiduelles est d'autant plus faible que la pression appliquée est plus élevée, ce qui explique la réduction de l'amplitude de la population correspondante en 1 et 10 µm.

L'amplitude de la fraction correspondant aux globules lipidiques émulsionnés augmente alors avec l'augmentation de la pression, à laquelle s'ajoute une réduction de la répartition granulométrique moyenne.

Des essais complémentaires, illustrés par la Figure 9, ont visé à évaluer le système mettant en oeuvre un ou deux passages à haute pression (250 MPa).

On note ainsi que l'influence de la pression est d'autant plus importante quand le procédé d'homogénéisation est constitué de deux passages successifs à très haute pression qu'un seul passage (générant ainsi une pression cumulée encore plus importante que la pression maximale atteignable en monopassage).

Par ces configurations d'homogénéisation à très haute pression, l'émulsion lipidique issue du cassage cellulaire est fortement stabilisée, ce qui facilite la suite des opérations de traitement de cette émulsion ainsi que sa mise en oeuvre en applications.

### Exemple 4. Réduction de la charge microbienne

Le traitement à très haute pression est évalué pour son potentiel de réduction de la contamination de la biomasse avant homogénéisation.

Le cisaillement engendré par la pression sur la valve de l'homogénéisateur, associée à la montée de la température, réduit significativement la charge microbienne de la biomasse et ainsi génère une force stérilisatrice.

Ainsi, le potentiel de décontamination est évalué à différent barème de pression sur le système continu d'ultra haute pression Stansted Fluid Power Ltd. 11300 (15 kW).

Le tableau suivant présente les résultats de charge microbienne obtenus dans ces conditions.

| | **Point initial** | **HHP 250 MPa** | **HHP 350 MPa** |
|---|---|---|---|
| **T°C sortie** | 8 | 72 | 91 |
| **germes totaux à 30°C** | 42000 | <10 | <10 |

L'homogénéisation à très haute pression permet une réduction significative de la charge microbiologique en permettant un abattement microbien quasi-totale proche de la stérilisation.

### Description des Figures

**FIGURE 1** : Evaluation du taux de cassage selon la pression (100 MPa / 150 MPa) et le nombre de passes.
**FIGURE 2** **:** Evaluation du taux de cassage selon la pression en une seule passe
**FIGURE 3** **:** Evaluation du taux de cassage selon la pression en deux passes successives
**FIGURE 4** **:** Evaluation du taux de cassage en fonction de la température du produit à l'alimentation

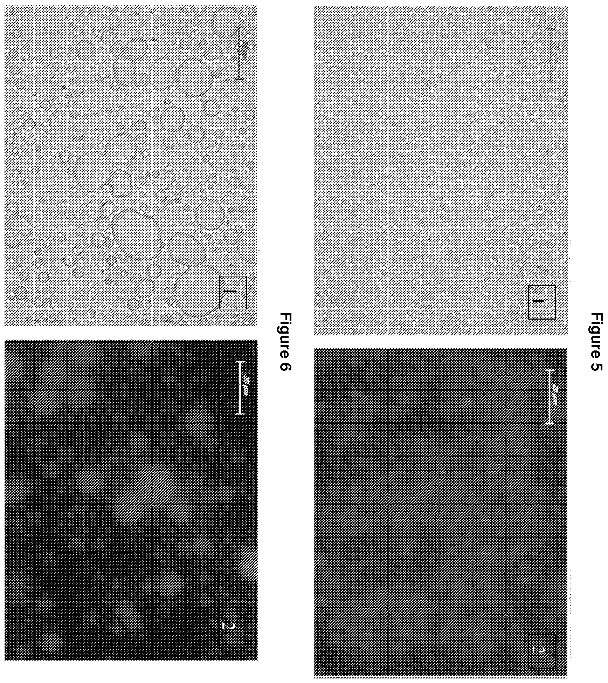

**FIGURE 5** **:** Observation microscopique de l'émulsion issue du broyage à billes (Grossissement x760, échelle : barre = 20 µm).
   1 - Lumière blanche
   2 - coloration au bleu de Nil et lumière fluorescente
**FIGURE 6** **:** Observation microscopique de l'émulsion issu du cassage HHP à 350 Mpa (Grossissement x760, échelle : barre = 20 µm).
   1 - Lumière blanche
   2 - coloration au bleu de Nil et lumière fluorescente
**FIGURE 7** **:** Analyse granulométrique de l'émulsion produite par broyage à billes ou par HHP relativement à la biomasse initiale
**FIGURE 8** **:** Analyse granulométrique de l'émulsion produite par HHP selon la pression appliquée
**FIGURE 9** **:** Analyse granulométrique de l'émulsion produite par HHP en 1 ou 2 passages à 250 MPa

## Revendications

1. Procédé de cassage de paroi cellulaire, à l'échelle industrielle, de cellules de microalgues du genre *Chlorella,* le cassage de paroi cellulaire étant conduit par homogénéisation à haute pression, **caractérisé en ce que** l'homogénéisation haute pression est conduite :
en au moins une passe à une pression entre 300 et 400 MPa, ou
en au moins deux passes successives à une pression comprise entre 150 et 300 MPa,
à une température d'alimentation comprise entre 4 et 40°C, et
à une biomasse de cellules de microalgues comprenant entre 15 et 50% en poids de matière sèche ;
de manière à garantir un taux de cassage de paroi cellulaire de plus de 80%, une granulométrie de l'émulsion produite inférieure à 1 µm et une diminution de la charge microbienne par un facteur 1000.

2. Procédé selon la revendication 1, **caractérisé en ce que** les microalgues du genre Chlorella sont choisies dans le groupe constitué de *Chlorella vulgaris, Chlorella sorokiniana* et *Chlorella protothecoides,* et sont plus particulièrement *Chlorella protothecoides.*

3. Procédé selon la revendication 1 ou 2, **caractérisé en ce que** le procédé garantit une charge microbienne de moins de 10 germes totaux par g d'émulsion.

4. Procédé selon l'une quelconque des revendications 1 à 3, **caractérisé en ce que** l'homogénéisation haute pression est conduite à une pression entre 300 et 400 MPa en une seule passe.

5. Procédé selon l'une quelconque des revendications 1 à 3, **caractérisé en ce que** l'homogénéisation haute pression est conduite à une pression comprise entre 150 et 250 MPa en deux, trois, quatre ou cinq passes successives.

6. Procédé selon la revendication 5, **caractérisé en ce que** l'homogénéisation haute pression est conduite à une pression comprise entre 150 et 250 MPa en deux passes successives.

7. Procédé selon l'une quelconque des revendications 1 à 3 et 5 à 6, **caractérisé en ce que** les pressions de deux passes successives peuvent être identiques ou différentes.

8. Procédé selon l'une quelconque des revendications 1-7, **caractérisé en ce que** la température d'alimentation est comprise entre 20 et 40°C.

9. Procédé selon l'une quelconque des revendications 1-8, **caractérisé en ce que** la biomasse de microalgues comprend au moins 10 % en poids sec de lipides, de préférence au moins 20, 30, 40, 50 ou 60 % en poids sec de lipides.

10. Procédé de préparation d'une farine de microalgues comprenant la production d'une biomasse de microalgues, le cassage de paroi cellulaire par un procédé selon l'une quelconque des revendications 1 à 9, et le séchage de la biomasse, en particulier par atomisation.

11. Utilisation du procédé de cassage de paroi cellulaire de microalgues selon l'une quelconque des revendications 1 à 9 pour la préparation d'une farine de microalgues.

## Patentansprüche

1. Verfahren zum Aufbrechen der Zellwand von Mikroalgenzellen der Gattung *Chlorella* in industriellem Maßstab, wobei das Aufbrechen der Zellwand durch Homogenisierung unter hohem Druck durchgeführt wird, **dadurch gekennzeichnet, dass** die Hochdruckhomogenisierung durchgeführt wird:
in wenigstens einer Passage unter einem Druck zwischen 300 und 400 MPa, oder
in wenigstens zwei aufeinanderfolgenden Passagen unter einem Druck zwischen 150 und 300 MPa,
bei einer Zufuhrtemperatur zwischen 4 und 40°C, und
mit einer Biomasse der Mikroalgenzellen umfassend zwischen 15 und 50 Gew.-% Trockenmasse;
um einen prozentualen Anteil an aufgebrochenen Zellwänden von mehr als 80%, eine Partikelgröße der gebildeten Emulsion von kleiner als 1 µm und eine Verringerung der mikrobiellen Last um einen Faktor 1000 sicherzustellen.

2. Verfahren gemäß Anspruch 1, **dadurch gekennzeichnet, dass** die Mikroalgen der Gattung *Chlorella* aus der Gruppe ausgewählt sind, bestehend aus *Chlorella vulgaris, Chlorella sorokiniana* und *Chlorella protothecoides,* und insbesondere *Chlorella protothecoides* sind.

3. Verfahren gemäß Anspruch 1 oder 2, **dadurch gekennzeichnet, dass** das Verfahren eine mikrobielle Last von weniger als 10 Gesamtkeimen pro Gramm Emulsion sicherstellt.

4. Verfahren gemäß einem der Ansprüche 1 bis 3, **dadurch gekennzeichnet, dass** die Hochdruckhomogenisierung unter einem Druck zwischen 300 und 400 MPa in einer einzigen Passage durchgeführt wird.

5. Verfahren gemäß einem der Ansprüche 1 bis 3, **dadurch gekennzeichnet, dass** die Hochdruckhomogenisierung unter einem Druck zwischen 150 und 250 MPa in zwei, drei, vier oder fünf aufeinanderfolgenden Passagen durchgeführt wird.

6. Verfahren gemäß Anspruch 5, **dadurch gekennzeichnet, dass** die Hochdruckhomogenisierung unter einem Druck zwischen 150 und 250 MPa in zwei aufeinanderfolgenden Passagen durchgeführt wird.

7. Verfahren gemäß einem der Ansprüche 1 bis 3 und 5 bis 6, **dadurch gekennzeichnet, dass** die Drücke der zwei aufeinanderfolgenden Passagen gleich oder verschieden sein können.

8. Verfahren gemäß einem der Ansprüche 1 bis 7, **dadurch gekennzeichnet, dass** die Zufuhrtemperatur zwischen 20 und 40°C beträgt.

9. Verfahren gemäß einem der Ansprüche 1 bis 8, **dadurch gekennzeichnet, dass** die Mikroalgenbiomasse wenigstens 10 Trockengew.-% Lipide, vorzugsweise wenigstens 20, 30, 40, 50 oder 60 Trockengew.-% Lipide umfasst.

10. Verfahren zur Herstellung eines Mikroalgenmehls, umfassend die Herstellung einer Mikroalgenbiomasse, das Aufbrechen der Zellwand mit einem Verfahren gemäß einem der Ansprüche 1 bis 9 und die Trocknung der Biomasse insbesondere durch Sprühtrocknung.

11. Verwendung des Verfahrens zum Aufbrechen der Zellwand von Mikroalgen gemäß einem der Ansprüche 1 bis 9 für die Herstellung eines Mikroalgenmehls.

## Claims

1. A process for breaking the cell wall, on an industrial scale, of cells of microalgae of the *Chlorella* genus, the cell-wall breaking being carried out by high-pressure homogenization, **characterized in that** the high-pressure homogenization is carried out:
in at least one pass at a pressure between 300 and 400 MPa, or
in at least two successive passes at a pressure between 150 and 300 MPa,
at a feed temperature between 4 and 40°C, and
with a biomass of microalgal cells comprising between 15% and 50% by weight of solids;
in such a way as to guarantee a degree of cell-wall breaking of more than 80%, a particle size of the emulsion produced of less than 1 µm and a decrease in microbial load by a factor of 1000.

2. The process as claimed in claim 1, **characterized in that** the microalgae of the *Chlorella* genus are chosen from the group consisting of *Chlorella vulgaris, Chlorella sorokiniana* and *Chlorella protothecoides,* and are more particularly *Chlorella protothecoides.*

3. The process as claimed in claim 1 or 2, **characterized in that** the process guarantees a microbial load of less than 10 total microorganisms per g of emulsion.

4. The process as claimed in any one of claims 1 to 3, **characterized in that** the high-pressure homogenization is carried out at a pressure between 300 and 400 MPa in a single pass.

5. The process as claimed in any one of claims 1 to 3, **characterized in that** the high-pressure homogenization is carried out at a pressure between 150 and 250 MPa in two, three, four or five successive passes.

6. The process as claimed in claim 5, **characterized in that** the high-pressure homogenization is carried out at a pressure between 150 and 250 MPa in two successive passes.

7. The process as claimed in any one of claims 1 to 3 and 5 to 6, **characterized in that** the pressures of two successive passes may be identical or different.

8. The process as claimed in any one of claims 1-7, **characterized in that** the feed temperature is between 20 and 40°C.

9. The process as claimed in any one of claims 1-8, **characterized in that** the microalgal biomass comprises at least 10% by dry weight of lipids, preferably at least 20%, 30%, 40%, 50% or 60% by dry weight of lipids.

10. A process for preparing a microalgal flour, which comprises production of a microalgal biomass, cell-wall breaking by means of a process as claimed in any one of claims 1 to 9, and drying of the biomass, in particular by spray drying.

11. The use of the process for breaking the cell wall of microalgae as claimed in any one of claims 1 to 9, for preparing a microalgal flour.
